# EUROPEAN PATENT SPECIFICATION

(11) **EP 0 843 212 B1**
(45) Date of publication and mention of the grant of the patent: **17.10.2001**
(21) Application number: 97203507.5
(22) Date of filing: 11.11.1997
(51) Int. Cl.: G03C 7/392, G03C 7/407, G03C 7/413, C07D 231/08

(54) **Photographic materials containing auxiliary developing agents and the use thereof**
Photographische Materialien, die Hilfsentwicklersubstanzen enthalten und Verwendung derselben
Matériaux photographiques contenant des agents auxiliaires de développement et l'usage desdits

(30) Priority: 14.11.1996 GB 9623709
(43) Date of publication of application: 20.05.1998
(73) Proprietor: EASTMAN KODAK COMPANY, Rochester, New York 14650 (US)
(72) Inventor: Tsoi, Siu C., Harrow, HA1 4TY (GB); Twist, Peter Jeffery, Harrow, HA1 4TY (GB)
(74) Representative: Haile, Helen Cynthia

(56) References cited:
- EP-A- 0 123 904
- EP-A- 0 679 943
- WO-A-92/10789
- US-A- 2 772 282
- US-A- 4 522 917
- US-A- 4 554 243

## Description

### Field of the Invention

This invention relates to auxiliary developing agents, photographic materials in which they are incorporated and a process for the development of photographic materials in which they are used.

### Background of the Invention

Pyrazolidone electron transfer agents have been previously described for use as auxiliary developing agents in black-and-white and colour photographic developers. It has been proposed to include these materials either in the developer solution or in the sensitized silver halide layer of a photographic film.

For example, US Patent 4,155,763 describes colour developing solutions comprising an aromatic amine colour developing agent and a 1-aryl-pyrazolidone having two substituents at the 4-position of the pyrazolidone. The advantages are said to include faster image dye formation and a more stable colour developing solution.

The English language abstract of Japanese application 62/178251 describes a colour developing solution comprising a para-phenylene colour developing agent and an auxiliary developing agent including, inter alia, 1-phenyl pyrazolidone. Image formation is described as highly sensitive and forming excellent gradation.

WO 92/10789 describes the colour development of an imagewise exposed photographic material in which the colour development is carried out in the presence of one or a combination of black-and-white silver halide developing agents such as pyrazolidones, for example: 1-(4-methoxyphenyl)-3-pyrazolidone, 1-(3,4-dimethoxyphenyl)-3-pyrazolidone and 1-phenyl-4-n-pentylpyrazolidone, which provide acceptable sensitometric results with reduced variability.

US Patent 2,772,282 describes the preparation of 4,4-dialkyl-3-pyrazolidones and their use as developing agents in photographic compositions.

US Patent 4,522,917 discloses a photographic material containing blocked 1-phenyl-3-pyrazolidones which are stable in storage and can release the photographically useful group at a controlled speed during development.

### Problem to be solved by the Invention

When an electron transfer agent such as a pyrazolidone is incorporated in the sensitized layer, residual amounts can season into the developer solution and can affect other films which did not contain this material and which were not designed to be processed in its presence.

This problem of the contamination of the developer solution by the presence of residual amounts of electron transfer agent when used for subsequent development has been solved by the use of a class of compounds which can accelerate development but are capable of becoming deactivated after a period of time in the developer solution.

### Summary of the Invention

According to the present invention there is provided a photographic recording material comprising a support and a photosensitive silver halide emulsion layer having in reactive association therewith an electron transfer agent comprising a 3-pyrazolidone compound substituted in the 1-position by an aromatic group, the aromatic group being substituted in the ortho, meta or para position by an -O-CO-R group, where R is a monovalent organic group containing up to 24 carbon atoms,
provided that the 3-pyrazolidone compound is not substituted in the 2-position by the group -CH₂-O-A, wherein A is a hydrolysable.group.

In a specific embodiment the emulsion layer has an image dye-forming coupler in reactive association therewith.

There is further provided a photographic development process which comprises developing the imagewise exposed photographic recording material in the presence of an electron transfer agent comprising the pyrazolidone described above.

Still further, this invention provides a photographic developer solution for the photographic recording material, the developer solution containing an electron transfer agent comprising the pyrazolidone described above.

The R group in the pyrazolidone preferably contains one or more aromatic rings. Particularly preferred compounds are those in which the R group comprises an aromatic ring which may be substituted, conveniently in the para position, with a group that imparts solubility in alkaline developer solution, such as methane sulphonamido. The aromatic ring may also be substituted by an electron donor (which has the effect of increasing the rate of deactivation) for example, hydroxy, amino, or hydroxyalkyl. Alternatively the aromatic ring may be substituted by an electron withdrawing group such as nitro, sulphamoyl, carbamoyl or nitrile. Such groups have the effect of slowing the rate of deactivation of the electron transfer agent.

The pyrazolidone is preferably substituted in the 4-position by one or more organic groups (R₂ and R₃) each having up to 12 carbon atoms, preferably two alkyl groups which may be the same or different each having up to 8 carbon atoms, preferably up to 6 carbon atoms, most preferably up to 3 carbon atoms.

### Advantageous Effect of the Invention

The compounds of the invention are useful as electron transfer agents but are deactivated after a period of time in the developer solution thereby preventing or reducing contamination of the developer solution by leaching of the electron transfer agent from a layer of photographic material into the developer solution.

### Brief Description of the Drawings

Figures 1-4 are graphical plots in which density of the image dye is plotted against the log of the exposure in the case of the development of a colour negative film. The plots show the results with and without an electron transfer agent, and with and without hydroxylamine, in the developer solution.
Figure 5 is a schematic representation of a photographic material in which an electron transfer agent is incorporated into a layer.

### Detailed Description of the Invention

Preferred pyrazolidone electron transfer agents for use in the present invention can be represented by either of the following general formulae, which show alternative tautomeric forms: 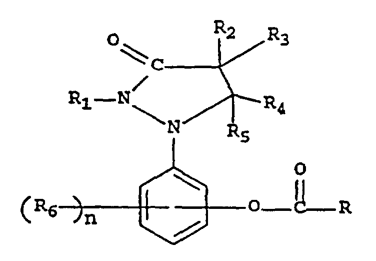 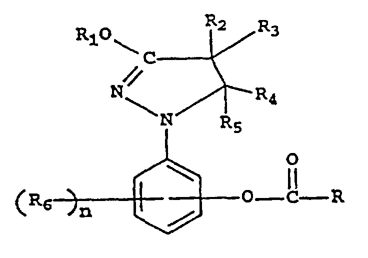
where R is a monovalent organic group having up to 24 carbon atoms, preferably from 1 to 12 carbon atoms and particularly from 1 to 6 carbon atoms,
R₁ is hydrogen,
R₂ and R₃ are independently hydrogen, substituted or unsubstituted alkyl having from 1 to 8 carbon atoms (such as hydroxyalkyl), carbamoyl, or substituted or unsubstituted aryl having from 6 to 10 carbon atoms,
R₄ and R₅ are independently hydrogen, substituted or unsubstituted alkyl having from 1 to 8 carbon atoms or substituted or unsubstituted aryl having from 6 to 10 carbon atoms,
R₆ can be hydrogen, but if not hydrogen, can be present in the ortho, meta or para positions of the benzene ring, with respect to the position attached to the nitrogen of the pyrazolidone, and can be halogen, substituted or unsubstituted alkyl or alkoxy having from 1 to 8 carbon atoms or sulphonamido and when there is more than one R₆ substituent, they can be the same or different or can be taken together to form a carbocyclic or heterocyclic ring including a benzene ring. Thus, n can be O or an integer from 1 to 4.

When R₂ and R₃ are alkyl, it is preferred that they have 1 to 3 carbon atoms. When R₂ and R₃ are each aryl, they are preferably phenyl. R₄ and R₅ are preferably hydrogen.

When R₆ is sulphonamido, it can be for example methanesulphonamido, ethanesulphonamido or toluenesulphonamido.

The electron transfer agent which is substituted in the aromatic ring with the -OCOR group can be any of those disclosed in EP-A-0 347 848A

It is preferred that the -OCOR group be attached to the ortho or para positions (with respect to the position attached to the nitrogen atom of the pyrazolidone ring).

The amount of electron transfer agent employed in this invention can be any concentration which is effective for the intended purpose. Suitable amounts are typically in the range from 0.04 to 8 mmol/m² (0.1 to 2 g/m²) preferably from 0.2 to 1.8 mmol/m² and more preferably from 0.5 to 1.5 mmol/m² of photographic recording material.

Especially preferred compounds ("ETAs") within the two tautomeric structural formulae are represented in the following Table.

| ETA No | R₂ | R₃ | R₆ |
|---|---|---|---|
| 1 | -H | -H | -H |
| 2 | -CH₃ | -H | -H |
| 3 | -CH(CH₃)₂ | -H | -H |
| 4 | -CH₃ | -CH₂OH | -H |
| 5 | -H | -H | p-CH₃ |
| 6 | -H | -H | p-OCH₃ |
| 7 | -CH₃ | -CH₂OH | p-CH₃ |
| 8 | -CH₃ | -CH₂OH | p-OCH₃ |

R is preferably an aromatic ring that can be substituted by a group that imparts solubility in alkaline developer solution, for example sulphonamido or methanesulphonamido, and optionally also by an electron donor (which has the effect of increasing the rate of deactivation) such as hydroxy, amino or hydroxyalkyl.

Alternatively the aromatic ring may be substituted with an electron withdrawing group such as nitro, sulphamoyl, carbamoyl or nitrile, which have the effect of slowing the rate of deactivation.

The electron transfer agents for use in the invention may be incorporated into black-and-white papers or films as auxiliary developing agents, or into colour negative paper or films where they may be used to lower process sensitivity, or they may be used to accelerate development in all of the elements noted the above. These types of papers and films are well known in the art, as described, for example, in Research Disclosure, Publication 38957, September 1996.

Preferably there is incorporated into the developer solution used in the development process of this invention, a catalyst such as hydroxylamine, in an effective amount for the deactivation of the electron transfer agent after a sufficient period has elapsed for the electron transfer agent to accelerate development. Thus the catalyst can be generally present in the developer solution in an amount of from 0.1 to 20g/l, preferably 0.5 to 10g/l, more preferably 2 to 5g/l.

In carrying out the process, it is preferred that the electron transfer agent be incorporated into a layer of the photographic recording material. However it is possible to provide the electron transfer agent in the developer solution. This is not preferred, however, because contamination of the developer solution with residual amounts of electron transfer agent is more likely to occur in such embodiments. When incorporated into the developer solution, the electron transfer agent is generally present in an amount of from 1 x 10⁻⁴ to 5 x 10⁻³ molar, preferably 3 x 10⁻⁴ to 1 x 10⁻³ molar.

The invention is illustrated by the following Examples.

### Example 1

### Synthesis of ETA 1

To a solution of p-nitrophenol (13.9g, 0.1mol) in dry tetrahydrofuran (200ml) was added triethylamine (11.0g, 0.11mol) at room temperature with stirring. The reaction mixture was cooled to about 10°C and benzoyl chloride (14.5g, 0.103 mol) was added dropwise to the above mixture at such a rate that the temperature did not rise above 15°C. After the addition was completed, the mixture was stirred at room temperature for 20 hours before pouring into a mixture of ice/water (1:1) and concentrated hydrochloric acid with rapid stirring. The white precipitate was collected by filtration and washed with water (1:1) under suction. After drying under vacuum over P₂O₅ at 45°C, a white solid was obtained as the required intermediate (1). Yield, 24.2g (99.5%).

This compound was used in the next stage of the synthesis without further purification.

A solution of intermediate (1) (5.0g, 20.5mmol) in dry tetrahydrofuran (250ml) was hydrogenated over 10% palladium on charcoal (0.5g) under 32 atmospheres of hydrogen. The hydrogenation was over 2.5 hours at room temperature. After removal of the catalyst, the solvent was rotary evaporated to dryness to give the required aniline (2) as an off-white solid. Yield 4.4g (100%). The aniline (2) was used in the next stage of the synthesis without further purification.

To a suspension of the aniline (2) (4.28g, 20 mmol) in a mixture of concentrated hydrochloric acid (23ml) and water (8ml) was added dropwise an ice-cold solution of sodium nitrite (1.5g, 21.8 mmol) in water (4.5 ml) at 0°C with rapid stirring. The addition was carried out over a period of 15 minutes and the mixture was then stirred at 0°C for a further 15 minutes. This mixture was poured into a solution of tin (II) chloride (16.8g, 88.7 mmol) in a mixture of concentrated hydrochloric acid (180ml) at room temperature with stirring. After stirring for about one hour, the precipitate was collected by filtration under suction. The crude material was triturated with acetonitrile (80ml), filtered and dried under vacuum to give the required hydrazine hydrochloride (3). Yield 3.54g, (67%). The hydrazine hydrochloride (3) was used in the preparation of ETA 1 without further purification.

To a suspension of the hydrazine hydrochloride (3) in dry pyridine (10ml) was added dropwise 3-chloropivaloyl chloride (0.59g, 3.78 mmol), at 5°C, over a period of 10 minutes. The mixture was stirred at about 5°C for a further one hour and then at room temperature for 22 hours. After pouring into a rapidly stirred mixture of ice/water (150ml) and concentrated hydrochloric acid (12ml) a pale yellow precipitate was formed. The solid was collected by filtration under suction and the damp solid dissolved in ethyl acetate (150ml). The organic solution was washed with water (100ml) and then brine (100ml). After drying over magnesium sulphate, the solvent was removed under reduced pressure and the resultant solid triturated with diethyl ether (50ml). The solid was collected by filtration under suction and then dried in vacuum. The required product, ETA 1, was obtained as a cream coloured solid. Yield, 0.92g (79%).

| | | | | |
|---|---|---|---|---|
| C₁₈H₁₈N₂O₃ | requires: | C, 69.66; | H, 5.85; | N, 9.03% |
| | found | C, 69.68; | H, 5.75; | N, 8.97% |

The synthetic route is illustrated as follows:-

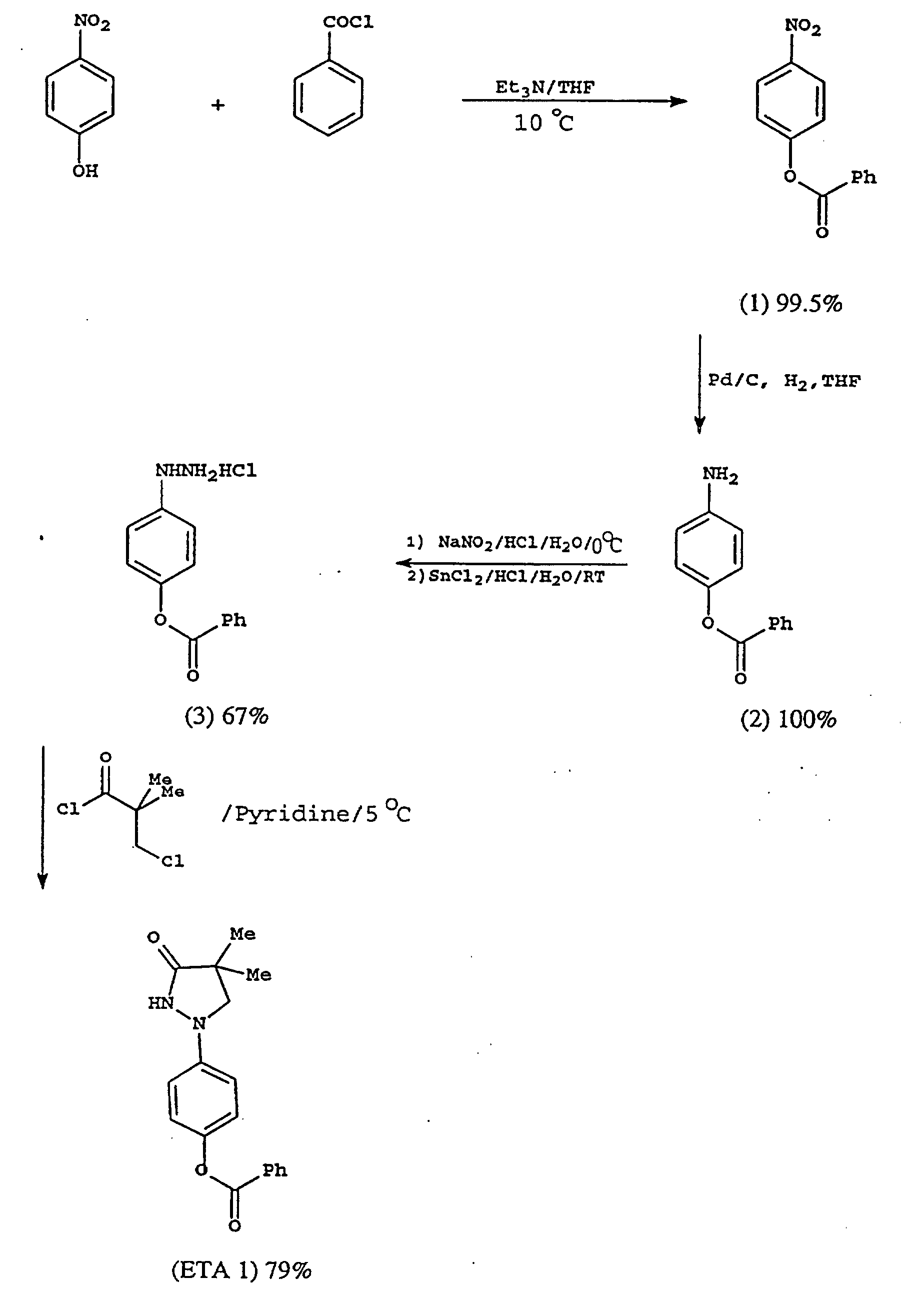

### Use of ETA 1 as an electron transfer agent in a colour development process.

A colour negative developer composition shown in Table 1 was made with and without hydroxylamine (HAS).

**Table 1**

| | |
|---|---|
| potassium carbonate (anhydrous) | 37.5g/l |
| sodium sulphite (anhydrous) | 4.25g/l |
| potassium iodide | 1.2mg/l |
| sodium bromide | 1.3g/l |
| hydroxylamine sulphate | 2.0g/l |
| anti-calcium sequestering agent | 6.5ml/l |
| colour developing agent | 4.5g/l |
| pH | 10.00 |

The anti-calcium sequestering agent was a 40% w/w aqueous solution of the pentasodium salt of diethylene triamine penta-acetic acid and the colour developing agent was 4-amino-3-methyl-N-ethyl-N-(hydroxyethyl)aniline sulphate.

An electron transfer agent of the structure shown as ETA 1 was examined as a development accelerator for a single layer colour negative material comprising a silver bromo-iodide emulsion (0.9g/m²) and a cyan image forming coupler (0.6g/m²). ETA 1 (0.15g/l) was dissolved in the developer shown in Table 1 but without any hydroxylamine sulphate (HAS) present.

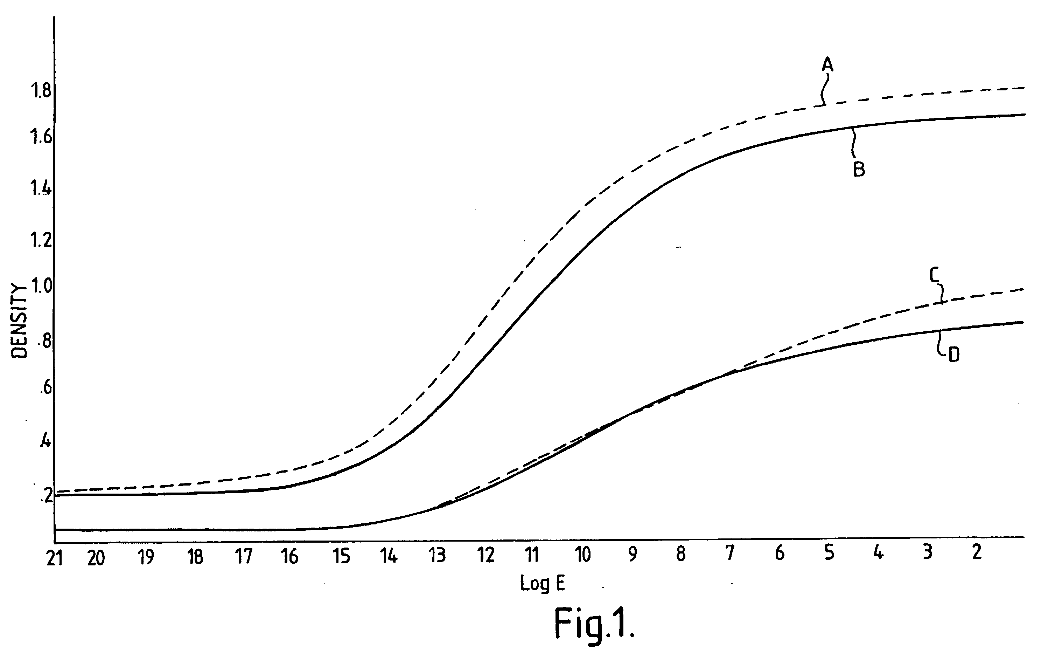

The result shown in Figure 1 was obtained. Curves A and B represent data from 2.5 minute development, with and without ETA 1 respectively. Curves C and D represent data from 1 minute development, with and without ETA respectively. It can be seen that there is a small acceleration in development after 2.5 minutes but very little after 1 minute.

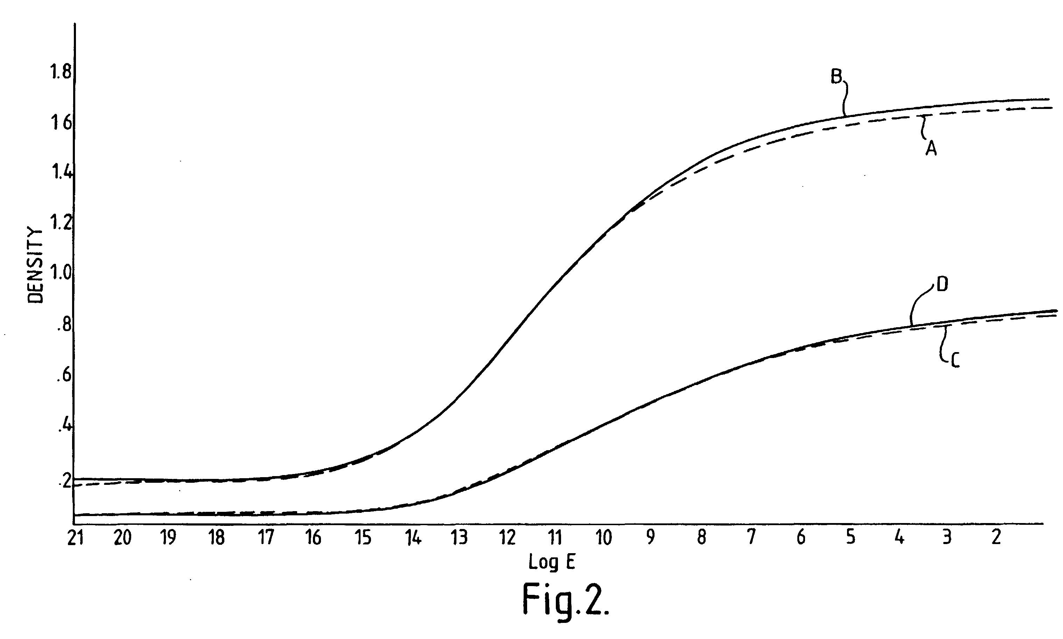

When hydroxylamine (HAS) was included in the developer solution, the small amount of acceleration disappeared as can be seen from Figure 2. Curves A, B, C and D in Figure 2 represent the same conditions as for Figure 1. The first film strips were processed 10 minutes after ETA 1 was added to the developer solution. It is apparent that within this time ETA 1 had degraded and was no longer acting as a development accelerator. It is also clear that the loss of ETA 1 was accelerated by the presence of HAS in the developer.

### Example 2

### Synthesis of ETA 2.

To a solution of ethyl 4-aminobenzoate (16.5g, 0.1mol) and N,N-dimethylaniline (13.3g, 0.11mol) in dry tetrahydrofuran (100ml) was added dropwise methane sulphonyl chloride (12.6g, 0.11mol) at room temperature with stirring. The mixture was then refluxed for 20 hours. After cooling to room temperature the mixture was poured into ice/water (1:1) and concentrated hydrochloric acid (20ml) with rapid stirring. The resultant solid (4) was collected and washed with water by filtration under suction. The damp solid was dissolved in a mixture of tetrahydrofuran (90ml) and ethanol (80ml). A solution of sodium hydroxide (12g, 0.3 mol) in water (80ml) was then added to the above solution and refluxed for 24 hours. After cooling to room temperature, the reaction mixture was poured into a mixture of ice/water (1:1) and concentrated hydrochloric acid (50ml) with rapid stirring. The white solid was collected by filtration under suction and then dried at 40°C over P₂O₅ to give the required product (5). Yield 19.5g, (91%).

This compound was used in the next stage of the synthesis without further purification.

A suspension of the acid (5) (14.3g, 66.5 mmol) in a mixture of thionyl chloride (11.5g, 96.6 mmol) and ethyl acetate (60ml) was refluxed for 24 hours. After cooling to room temperature and then in an ice/water bath for a further 15 minutes, a white solid was formed and was collected and washed with diethyl ether by filtration under suction. The crude product was dissolved in dry tetrahydrofuran (60ml) and added dropwise to a mixture of p-nitrophenol (9.23g, 66.4mmol) and triethylamine (7.3g, 72mmol) in dry tetrahydrofuran (100ml) at 5°C with stirring over a period of 30 minutes. The reaction mixture was stirred at room temperature for about 24 hours and then poured into a mixture of ice/water (1.51) and concentrated hydrochloric acid with rapid stirring. The resultant solid was collected and washed with water by filtration under suction. The product was obtained as a creamy white solid after drying under vacuum over P₂O₅ at 40°C. Yield 17.8g (79%).

Product (6) (17.5g 52.1mmol) in tetrahydrofuran (500ml) was hydrogenated over 10% palladium on charcoal (1.7g) at 35°C under 34 atmospheres of hydrogen for 24 hours. After removal of the catalyst by filtration the filtrate was treated with hydrochloric acid gas with cooling in an ice/water bath. A white precipitate was formed and was collected and washed with cold tetrahydrofuran under suction. After drying under vacuum, the amine hydrochloride (7) was isolated as a white solid. Yield 17.5g (98%).

To a suspension of the finely powdered amine hydrochloride (7) (1g, 2.92 mmol) in a mixture of concentrated hydrochloric acid (10ml) and water (3ml) was added dropwise an ice-cold solution of sodium nitrite (0.25g, 3.6mmol) in water (1ml) at 5°C with rapid stirring. After the addition was completed, the mixture was stirred at about 10°C for a further 10 minutes after which a complete solution was observed. The above solution was added dropwise to a solution of tin (II) chloride (2.3g, 12.3mmol) in a mixture of concentrated hydrochloric acid (15ml) and water (35ml) at about 10°C with rapid stirring. A white precipitate separated and was collected by filtration under suction. The solid was washed with cold hydrochloric acid (3 Normal) dried under vacuum over P₂O₅ at 40°C to give an off-white solid (1g). This solid was triturated with tetrahydrofuran (35ml) filtered and dried under vacuum to give the required hydrazine hydrochloride (8) as a tan coloured solid. Yield 0.85g (81%).

To a suspension of the hydrazine hydrochloride (8) (0.85g 2.38mmol) in dry pyridine (10ml) was added dropwise 2-chloropivaloyl chloride (0.38g, 2.45mmol) with stirring. The reaction mixture was stirred at 5°C for a further 2 hours and then at room temperature for 22 hours. After pouring into a rapidly stirred mixture of concentrated hydrochloric acid (12ml) and ice/water (120ml), a precipitate was formed and was collected and washed with water by filtration under suction. A light brown solid (0.9g) was obtained after drying under vacuum over P₂O₅. The crude product was purified by column chromatography (160g silica gel eluted with ethyl acetate) and the fractions containing the required product were combined and concentrated under reduced pressure. The residual gum was dissolved in a minimum of tetrahydrofuran and added dropwise to rapidly stirred petrol (60 to 80°C) (200ml). The precipitate was collected by filtration under suction and then dried under vacuum to give the required product (ETA 2) as a peach coloured solid. Yield 0.77g (80%).
1H-NMR (DMSO-d6):1.05 (s, 6H), 3.15 (s,3H), 3.7(s,2H), 7.05 (d,2H), 7.15 (d,2H), 7.35 (d,2H), 8.05 (d,2H), 10.15(s,1H), and 10.4 (s, 1H). M/z:403.

This synthetic route is illustrated as follows:-

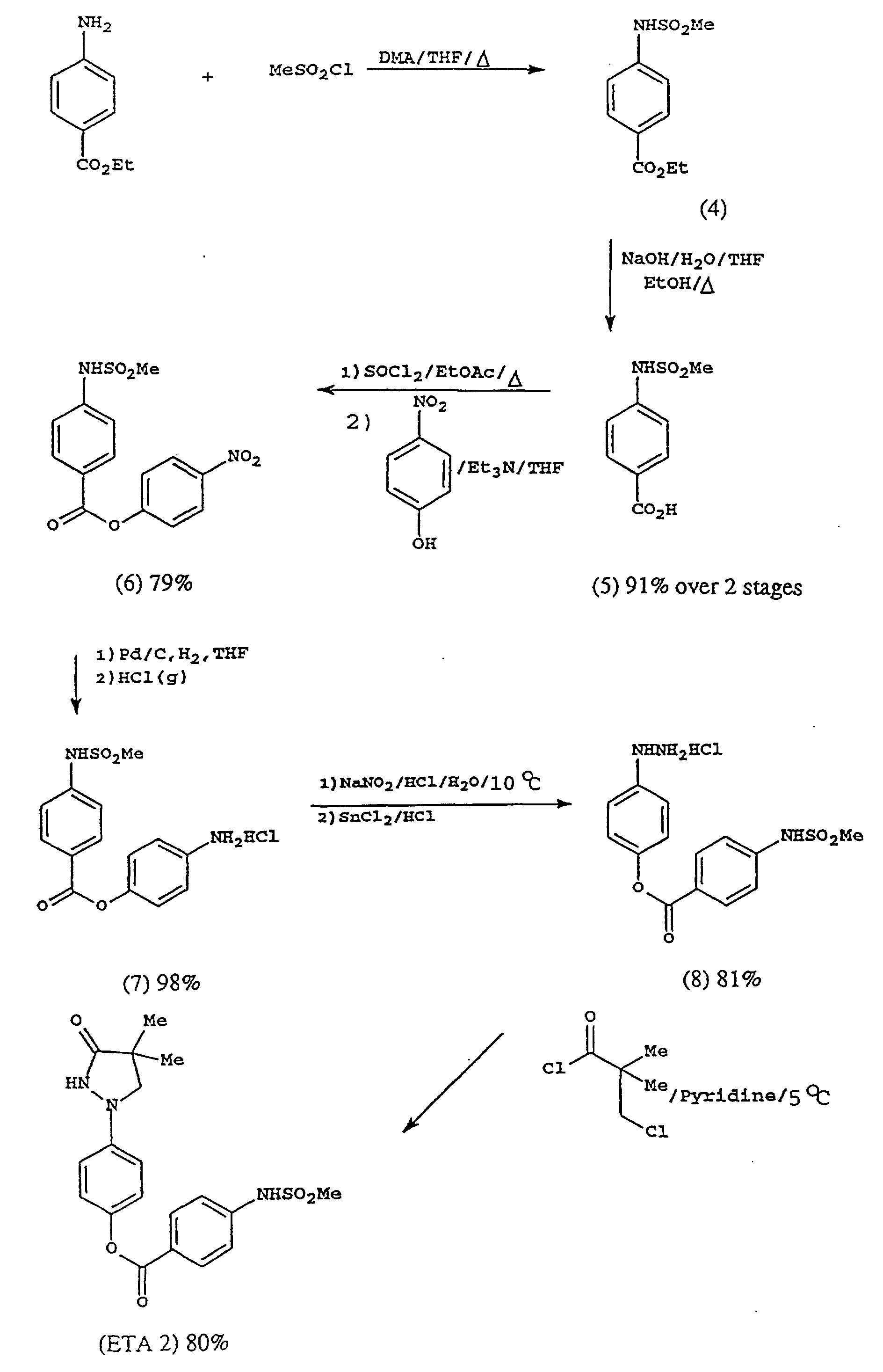

### Use of compound ETA 2 as an electron transfer agent in a colour development process.

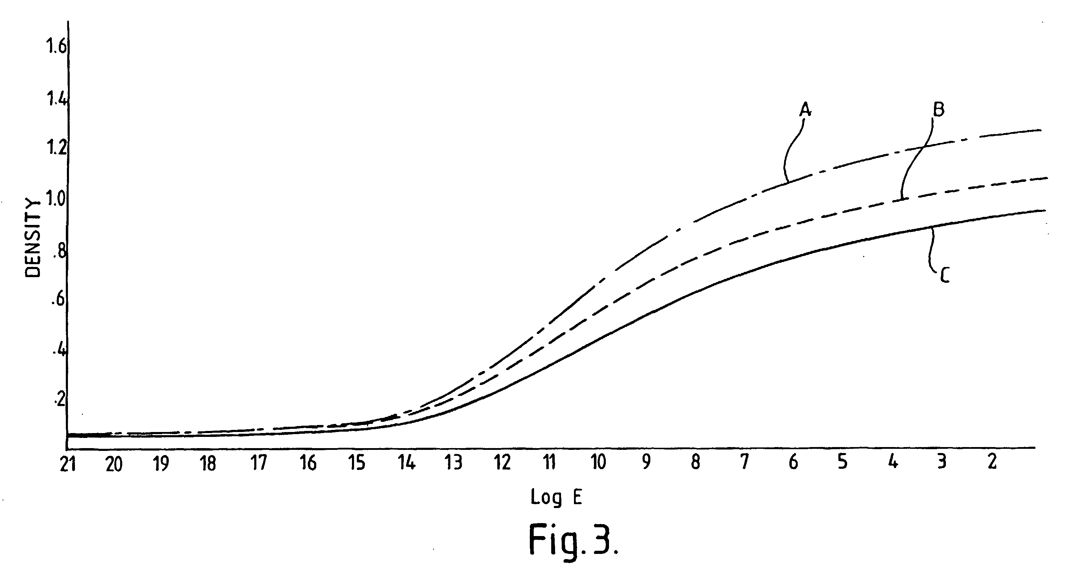

ETA 2 was evaluated in the same element described in Example 1. The result is shown in Figure 3 for 1 minute development time in the developer shown in Table 1 with varying amounts of ETA 2 and without hydroxylamine present. Curve A represents 0.2g/l ETA 2, Curve B represents 0.1g/l ETA 2 and Curve C represents no ETA 2. Here it can be seen that there is a significant acceleration of development effected by ETA 2.

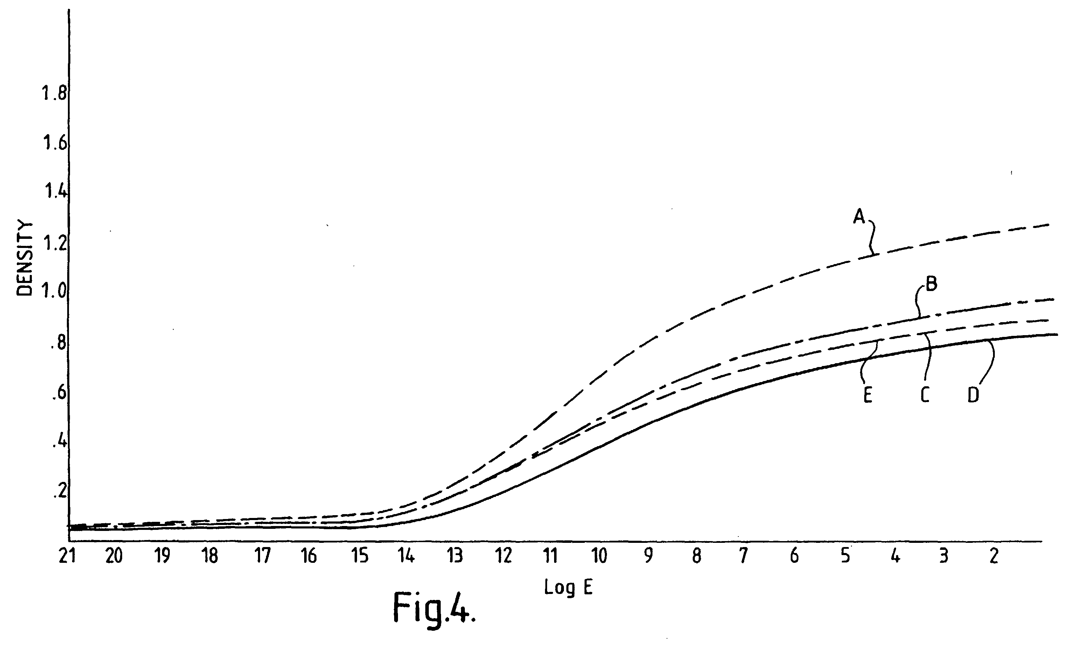

Hydroxylamine was added to the developer solution and film strips were processed as soon as possible afterwards and it can be seen from Figure 4 that in about 1.5 minutes there was a significant reduction in the amount of development acceleration. Curve A represents 0.2g/l ETA 2 and no HAS. Curve B represents 0.2g/l ETA 2 and 1 minute development after addition of HAS (2g/l, 1 minute 22 seconds later). Curve C represents 0.2g/l ETA 2 and 1 minute development after addition of HAS (2g/l, 1 hour 16 minutes development after addition). Curve D represents the use of HAS (2g/l) and no ETA. A developer without either ETA 2 and HAS (shown by Curve E) gave the same results shown as Curve C. Film strips were processed over a period of time and the activity of the compound ETA 2 gradually diminished until it was almost equivalent to the control without any ETA 2.

These results demonstrate that the electron transfer agent ETA 2 is a development accelerator and that in the presence of hydroxylamine it loses its accelerating ability within a period of several minutes.

### Example 3

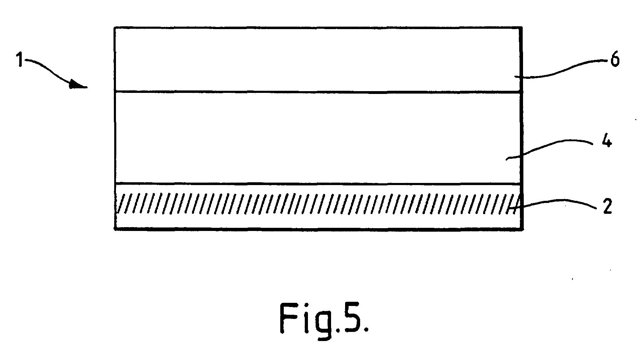

Referring to Figure 5, a photographic recording material 1 comprises antihalation support 2, emulsion layer 4 containing a colour coupler, silver halide and ETA 2 and gelatin overcoat 6.

The coupler was a cyan coupler having the structure labelled C1 below, coated at 0.6g/m². The silver halide was a 400 speed silver bromo-iodide (4% iodide) T-grain emulsion coated at 0.9g/m². Gelatin was coated at 2.7g/m² in emulsion layer 4 and at 1.0g/m² in overcoat layer 6. ETA 2 was coated at 0.1g/m².

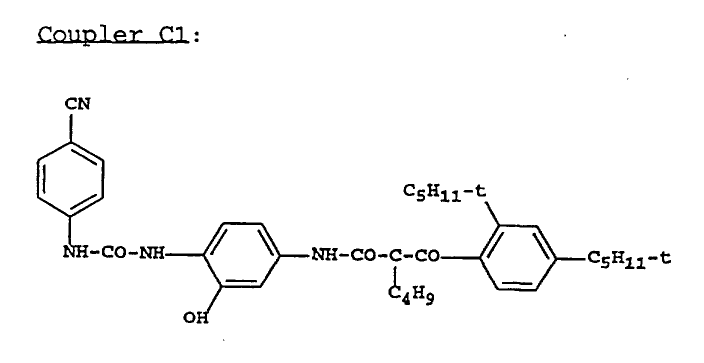

ETA 2 was stable in the coating until the element was processed in the developer solution shown in Table 1 above. Development of the silver bromo-iodide commenced and was accelerated by ETA 2. In about one minute, most ETA 2 had diffused out of the layer and into the developer solution and in the next few minutes ETA 2 was deactivated by the action of the hydroxylamine in the developer solution.

In order for the effective working of the invention, the rate of deactivation of the ETA must not be so high as to prevent it acting as a development accelerator which will occur primarily during the first minute of the development. It is clear that compound ETA 2 has achieved this objective since it lost about 70% of its accelerating ability within one minute and 22 seconds. Thus, during this period and primarily during the first minute before the ETA diffuses out of the layer, it is able to act as a development accelerator.

It is also clear that a range of ETA types based on 1-phenyl-3-pyrazolidone with a hydrolysable ester group on the phenyl ring but with different substituents from those in ETA 2 (to either increase or decrease the accelerating ability or to increase or decrease its rate of deactivation) can be employed in this invention.

The advantages of the electron transfer agents used in the preceding Examples are that:
i. they can be incorporated into photographic layers without causing seasoning effects,
ii. their decomposition and hence deactivation is accelerated by hydroxylamine.

## Claims

1. A photographic recording material comprising a support and a photosensitive silver halide emulsion layer having in reactive association therewith an electron transfer agent comprising a 3-pyrazolidone substituted in the 1-position by an aromatic group, the aromatic group being substituted in the ortho, meta or para position by an -O-CO-R group, where R is a monovalent organic group containing up to 24 carbon atoms,
provided that the 3-pyrazolidone compound is not substituted in the 2-position by the group -CH₂-O-A, wherein A is a hydrolysable group.

2. A recording material as claimed in claim 1 wherein the R group contains an aromatic ring.

3. A recording material as claimed in either of the preceding claims wherein the pyrazolidone is substituted in the 4-position by one or more organic groups, R₂ and R₃, each having up to 12 carbon atoms.

4. A recording material as claimed in claim 3 wherein R₂ and R₃ are alkyl groups which may be the same or different each having up to 6 carbon atoms.

5. A recording material as claimed in any one of the preceding claims having either of the general formulae: 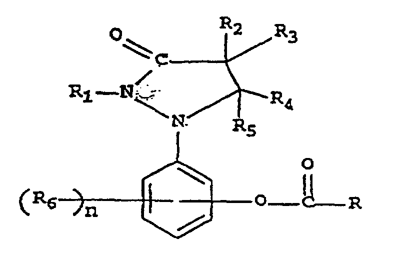 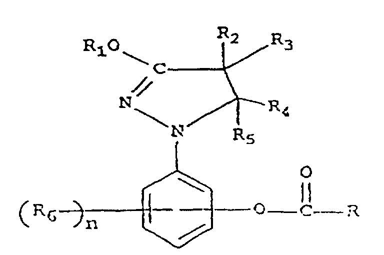
where R is a monovalent organic group having up to 24 carbon atoms,
R₁ is hydrogen,
R₂ and R₃ are independently hydrogen, substituted or unsubstituted alkyl having from 1 to 8 carbon atoms, carbamoyl, or substituted or unsubstituted aryl having from 6 to 10 carbon atoms,
R₄ and R₅ are independently hydrogen, substituted or unsubstituted alkyl having from 1 to 8 carbon atoms or substituted or unsubstituted aryl having from 6 to 10 carbon atoms,
R₆ can be hydrogen but if not hydrogen, can be present in the ortho, meta or para positions of the benzene ring, with respect to the position attached to the nitrogen of the pyrazolidone, and can be halogen, substituted or unsubstituted alkyl or alkoxy having from 1 to 8 carbon atoms or sulphonamido and when there is more than 1 R₆ substituent, they can be the same or different or can be taken together to form a carbocyclic or heterocyclic ring including a benzene ring and n is 0 to 4.

6. A recording material as claimed in claim 5 wherein R is a monovalent organic group having 1 to 6 carbon atoms.

7. A recording material as claimed in any one of the preceding claims wherein the emulsion layer has an image dye-forming coupler in reactive association therewith.

8. A recording material as claimed in any of the preceding claims wherein the electron transfer agent is present in an amount of from 0.04 to 8 mmol/m².

9. A photographic development process comprising developing an imagewise exposed photographic recording material comprising a support and a photosensitive silver halide emulsion, in the presence of an electron transfer agent comprising a pyrazolidone as defined in any one of claims 1 to 6.

10. A photographic development process as claimed in claim 9 wherein the developing is carried out with a developer solution comprising a catalyst in an amount effective for the deactivation of the electron transfer agent.

11. A photographic development process as claimed in claim 10 wherein the catalyst is hydroxylamine.

12. A process as claimed in any one of claims 9 to 11 wherein the catalyst is present in an amount of from 0.1 to 20g/l.

13. A photographic developer solution for a photographic recording material comprising a support and a photosensitive silver halide emulsion, said developer solution containing an electron transfer agent comprising a pyrazolidone as defined in any one of claims 1 to 6.

14. A solution as claimed in claim 13 wherein the electron transfer agent is present in an amount of from 1 x 10⁻⁴ to 5 x 10⁻³ molar.

## Patentansprüche

1. Fotografisches Aufzeichnungs-Material mit einem Träger und einer foto-sensitiven Silberhalogenid-Emulsionsschicht, die in reaktiver Zuordnung ein Elektronen-Übertragungsmittel enthält, das ein 3-Pyrazolidon umfaßt, das in der 1-Position durch eine aromatische Gruppe substituiert ist, wobei die aromatische Gruppe in der ortho-, meta- oder para-Position durch eine -O-CO-R-Gruppe substituiert ist, worin R eine monovalente, organische Gruppe mit bis zu 24 Kohlenstoffatomen ist,
wobei gilt, daß die 3-Pyrazolidon-Verbindung nicht in der 2-Position durch die Gruppe -CH₂-O-A substituiert ist, in der A eine hydrolysierbare Gruppe ist.

2. Aufzeichnungs-Material nach Anspruch 1, worin die Gruppe R einen aromatischen Ring enthält.

3. Aufzeichnungs-Material nach einem der vorstehenden Ansprüche, worin das Pyrazolidon in der 4-Position durch eine oder mehrere organische Gruppen, R₂ und R₃, substituiert ist, die jeweils bis zu 12 Kohlenstoffatome aufweisen.

4. Aufzeichnungs-Material nach Anspruch 3, worin R₂ und R₃ Alkylgruppen sind, die gleich oder verschieden sein können und jeweils bis zu 6 Kohlenstoffatome aufweisen.

5. Aufzeichnungs-Material nach einem der vorstehenden Ansprüche mit einer der allgemeinen Formeln: 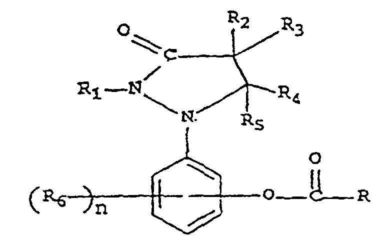 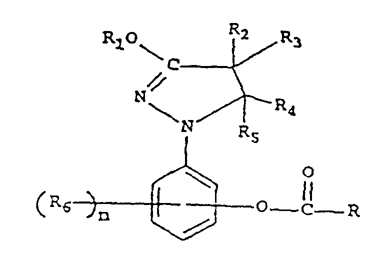
worin R eine monovalente, organische Gruppe mit bis zu 24 Kohlenstoffatomen ist,
R₁ für Wasserstoff steht,
R₂ und R₃ unabhängig voneinander stehen für Wasserstoff, substituiertes oder unsubstituiertes Alkyl mit 1 bis 8 Kohlenstoffatomen, Carbamoyl, oder substituiertes oder unsubstituiertes Aryl mit 6 bis 10 Kohlenstoffatomen,
R₄ und R₅ unabhängig voneinander stehen für Wasserstoff, substituiertes oder unsubstituiertes Alkyl mit 1 bis 8 Kohlenstoffatomen, oder substituiertes oder unsubstituiertes Aryl mit 6 bis 10 Kohlenstoffatomen,
R₆ für Wasserstoff stehen kann, jedoch dann, wenn R₆ nicht für Wasserstoff steht, in den ortho-, meta- oder para-Positionen des Benzolringes, bezüglich der Position der Bindung zu dem Stickstoff des Pyrazolidons, vorliegen kann, und bestehen kann aus Halogen, substituiertem oder unsubstituiertem Alkyl oder Alkoxy mit 1 bis 8 Kohlenstoffatomen oder Sulfonamido, wobei, wenn mehr als 1 R₆-Substituent vorliegt, diese gleich oder verschieden sein können oder zusammengenommen werden können, unter Bildung eines carbozyklischen oder heterozyklischen Ringes, einschließlich eines Benzolringes, und worin n gleich 0 bis 4 ist.

6. Aufzeichnungs-Material nach Anspruch 5, worin R eine monovalente, organische Gruppe mit 1 bis 6 Kohlenstoffatomen ist.

7. Aufzeichnungs-Material nach einem der vorstehenden Ansprüche, worin die Emulsionsschicht einen, einen Bild-Farbstoffbildenden Kuppler in reaktiver Zuordnung enthält.

8. Aufzeichnungs-Material nach einem der vorstehenden Ansprüche, worin das Elektronen-Übertragungsmittel in einer Menge von 0,04 bis 8 mMol/m² vorliegt.

9. Fotografischer Entwicklungs-Prozeß, der umfaßt die Entwicklung eines bildweise exponierten, fotografischen Aufzeichnungs-Materials mit einem Träger und einer foto-sensitiven Silberhalogenid-Emulsion, in Gegenwart eines Elektronen-Übertragungsmittels, das ein Pyrazolidon, wie in einem der Ansprüche I bis 6 definiert, umfaßt.

10. Fotografischer Entwicklungs-Prozeß nach Anspruch 9, worin die Entwicklung mit einer Entwickler-Lösung durchgeführt wird, die einen Katalysator in einer Menge enthält, die wirksam für die Deaktivierung des Elektronen-Übertragungsmittels ist.

11. Fotografischer Entwicklungs-Prozeß nach Anspruch 10, worin der Katalysator Hydroxylamin ist.

12. Verfahren nach einem der Ansprüche 9 bis 11, worin der Katalysator in einer Menge von 0,1 bis 20 g/l vorliegt.

13. Fotografische Entwickler-Lösung für ein fotografisches Aufzeichnungs-Material mit einem Träger und einer foto-sensitiven Silberhalogenid-Emulsion, wobei die Entwickler-Lösung ein Elektronen-Übertragungsmittel enthält, das ein Pyrazolidon, wie in einem der Ansprüche 1 bis 6 definiert, enthält.

14. Lösung nach Anspruch 13, worin das Elektronen-Übertragungsmittel in einer Menge vorliegt, die 1 x 10⁻⁴ bis 5 x 10⁻³ molar ist.

## Revendications

1. Produit d'enregistrement photographique comprenant un support et une couche d'émulsion aux halogénures d'argent photosensible à laquelle est associé un agent de transfert d'électrons comprenant une 3-pyrazolidone substituée en position 1 par un groupe aromatique, le groupe aromatique étant substitué en position ortho, méta ou para par un groupe -O-CO-R, où R est un groupe organique monovalent contenant jusqu'à 24 atomes de carbone,
à la condition que le composé 3-pyrazolidone ne soit pas substitué en position 2 par le groupe -CH₂-O-A, où A est un groupe hydrolysable.

2. Produit d'enregistrement selon la revendication 1, dans lequel le groupe R contient un cycle aromatique.

3. Produit d'enregistrement selon l'une ou l'autre des revendications précédentes, dans lequel la pyrazolidone est substituée en position 4 par un ou plusieurs groupes organiques, R₂ et R₃, chacun ayant jusqu'à 12 atomes de carbone.

4. Produit d'enregistrement selon la revendication 3, dans lequel R₂ et R₃ sont des groupes alkyle pouvant être identiques ou différents, chacun ayant jusqu'à 6 atomes de carbone.

5. Produit d'enregistrement selon l'une quelconque des revendications précédentes représentées par l'une ou l'autre des formules générales suivantes : 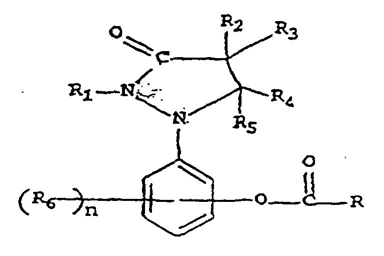 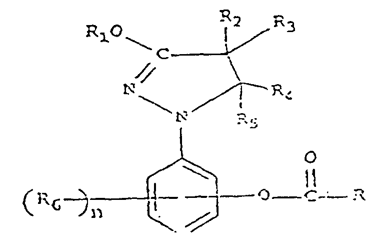
où R est un groupe organique monovalent ayant jusqu'à 24 atomes de carbone,
R₁ est l'hydrogène,
R₂ et R₃ sont indépendamment l'hydrogène, un groupe alkyle substitué ou non de 1 à 8 atomes de carbone, un groupe carbamoyle ou aryle substitué ou non de 6 à 10 atomes de carbone,
R₄ et R₅ sont indépendamment l'hydrogène, un groupe alkyle substitué ou non de 1 à 8 atomes de carbone, ou un groupe aryle substitué ou non de 6 à 10 atomes de carbone,
R₆ peut être l'hydrogène, mais si ce n'est pas l'hydrogène, il peut être présent en position ortho, méta ou para du cycle benzénique par rapport à la position rattachée à l'atome d'azote de la pirazolidone ; R₆ peut être un halogène, un groupe alkyle ou alcoxy substitué ou non de 1 à 8 atomes de carbone ou un groupe sulfonamido et lorsqu'il y a plus d'un substituant R₆, ils peuvent être identiques ou différents ou ensemble, former un carbocycle ou un hétérocycle comprenant un cycle benzénique et n est de 0 à 4.

6. Produit d'enregistrement selon la revendication 5, dans lequel R est un groupe organique monovalent de 1 à 6 atomes de carbone.

7. Produit d'enregistrement selon l'une quelconque des revendications précédentes, dans lequel la couche d'émulsion comprend un coupleur formateur de colorant d'image en association réactive avec cette dernière.

8. Produit d'enregistrement selon l'une quelconque des revendications précédentes, dans lequel l'agent de transfert d'électrons est présent en une quantité comprise entre 0,04 et 8 mmoles/m².

9. Procédé de développement photographique comprenant le développement d'un produit d'enregistrement photographique exposé en conformité avec l'image comprenant un support et une émulsion aux halogénures d'argent photosensible, en présence d'un agent de transfert d'électrons comprenant une pirazolidone telle que définie dans l'une quelconque des revendications 1 à 6.

10. Procédé de développement photographique selon la revendication 9, dans lequel le développement est mis en oeuvre en utilisant une solution de développement comprenant un catalyseur en quantité suffisante pour désactiver l'agent de transfert d'électrons.

11. Procédé de développement photographique selon la revendication 10, dans lequel le catalyseur est l'hydroxylamine.

12. Procédé selon l'une quelconque des revendications 9 à 11, dans lequel on utilise une quantité de catalyseur comprise entre 0,1 et 20 g/l.

13. Solution de développement photographique pour produit d'enregistrement photographique comprenant un support et une émulsion aux halogénures d'argent photosensible, ladite solution de développement contenant un agent de transfert d'électrons comprenant une pirazolidone telle que définie dans l'une quelconque des revendications 1 à 6.

14. Solution selon la revendication 13, dans laquelle on utilise une quantité d'agent de transfert d'électrons comprise entre 1 x 10⁻⁴ et 5 x 10⁻³ moles/l.
